# EUROPÄISCHE PATENTSCHRIFT

(11) **EP 1 515 932 B1**
(45) Veröffentlichungstag und Bekanntmachung des Hinweises auf die Patenterteilung: **16.06.2010**
(21) Anmeldenummer: 03757050.4
(22) Anmeldetag: 10.06.2003
(51) Int. Cl.: C07C 29/149, C07C 31/20

(54) **VERFAHREN ZUR HERSTELLUNG VON 1,4- BUTANDIOL**
METHOD FOR THE PRODUCTION OF 1,4- BUTANEDIOL
PROCEDE DE PRODUCTION DE 1,4-BUTANEDIOL

(30) Priorität: 11.06.2002 DE 10225926
(43) Veröffentlichungstag der Anmeldung: 23.03.2005
(73) Patentinhaber: BASF SE, 67056 Ludwigshafen (DE)
(72) Erfinder: HESSE, Michael, 67549 Worms (DE); SCHLITTER, Stephan, 67117 Limburgerhof (DE); BORCHERT, Holger, 67591 Offstein (DE); SCHUBERT, Markus, 67063 Ludwigshafen (DE); RÖSCH, Markus, 55276 Oppenheim (DE); BOTTKE, Nils, 68165 Mannheim (DE); FISCHER, Rolf-Hartmuth, 69121 Heidelberg (DE); WECK, Alexander, 67251 Freinsheim (DE); WINDECKER, Gunther, 67067 Ludwigshafen (DE); HEYDRICH, Gunnar, 67117 Limburgerhof (DE)
(86) Internationale Anmeldenummer: PCT/EP2003/006062
(87) Internationale Veröffentlichungsnummer: WO 2003/104174

(56) Entgegenhaltungen:
- WO-A-97/43234
- US-A- 5 196 602

## Beschreibung

Die vorliegende Erfindung betrifft ein Verfahren zur Herstellung von gegebenenfalls alkylsubstituiertem Butandiol durch katalytische Hydrierung in der Gasphase von Substraten, die ausgewählt sind aus der Gruppe bestehend aus Derivaten der Maleinsäure und Bernsteinsäure sowie diesen Säuren selbst. Unter Derivaten werden im Rahmen der vorliegenden Erfindung Anhydride verstanden, die ebenso wie die Säuren einen oder mehrere Alkylsubstituenten aufweisen können.

Die an sich bekannte Hydrierung von MSA führt über die Zwischenstufe Bernsteinsäureanhydrid (BSA) zunächst zu γ-Butyrolacton (GBL). Danach führt die weitere Hydrierung zu Tetrahydrofuran (THF), n-Butanol (BuOH) und/oder n-Butan. GBL steht mit BDO in einem Gleichgewicht, das durch geeignete Massnahmen zum grossen Teil auf die Seite des Butandiols verschoben werden kann. Butandiol kann jedoch ebenso leicht wie GBL durch sogenannte Überhydrierung zu Butanol und Butan abreagieren; Cyclisierung des Butandiol führt zu THF. Diese Produkte können nicht wieder in BDO bzw. GBL überführt werden. Wird BDO als Produkt gewünscht, muss die Bildung vor allem des THF vermieden werden.

Die Gasphasenhydrierung von gereinigtem Maleinsäureanhydrid (MSA) zu Butyrolacton (GBL) und die Umsetzung von gereinigtem GBL zu BDO sind zwei seit vielen Jahren bekannte Reaktionen. Zur Durchführung dieser beiden katalytischen Reaktionen sind in der Literatur zahlreiche Katalysatorsysteme beschrieben. Je nach Zusammensetzung der Katalysatoren und der gewählten Reaktionsparameter werden mit derartigen Katalysatoren unterschiedliche Produktverteilungen erreicht. Verfahren zur direkten Herstellung von Butandiol ausgehend von MSA sind ebenfalls bereits bekannt.

Sollen GBL und BDO hergestellt werden, die Alkylsubstituenten aufweisen, so bietet es sich an, von den vorstehend genannten Edukten auch die entsprechenden alkylsubstituierten Spezies zu verwenden.

Die bei der Hydrierung von MSA zu einem der vorstehend erwähnten Produkte verwendeten Katalysatoren sind, insbesondere bei älteren Verfahren, häufig chromhaltig. Dies findet seinen Niederschlag in der Patentliteratur, in der sich eine große Anzahl von Patenten und Patentanmeldungen findet, die chromhaltige Katalysatoren für die Hydrierreaktion offenbaren, wobei die Hydrierung in den meisten Fällen auf MSA als Edukt beschränkt ist.

Die nachfolgenden Dokumente beschreiben die Verwendung chromhaltiger Katalysatoren bei der Hydrierung von MSA.

In der EP 0 322 140-A1 wird ein kontinuierliches Verfahren zur Herstellung von Tetrahydrofuran (THF) und zur gleichzeitigen Herstellung von THF und GBL durch Gasphasenhydrierung von MSA und BSA offenbart. Der beanspruchte Katalysator enthält Kupfer, Zink und Aluminium und ein weiteres Element der Gruppe IIA, IIIA, VA, VIII, IIIB bis VIIB, der Lanthan und Actinium-Reihe, bzw. Ag und Au. Bei 40 bar können mit diesen Katalysatorsystemen ausgehend von Rein-MSA THF-Ausbeuten von 90 - 95 % erhalten werden, bei einem Druck von ca. 20 bar lassen sich Gemische von GBL und THF erhalten.

In den US 4,965,378 und US 5,072,009 kommt ein ähnlicher Katalysator zur Anwendung, der jedoch zusätzlich Si, Ge, Sn und Pb enthalten kann. Durch die Anwendung solcher Katalysatoren entstehen hohe Mengen THF (95% bis 31,4%), das nicht zu Butyrolacton oder Butandiol umgesetzt werden kann.

Die EP-A 0 404 408 offenbart einen Katalysator für die MSA-Hydrierung, dessen katalytisch aktives Material im wesentlichen dem Material der US 5,072,009 entspricht. Es wird auf einem Träger fixiert als Schalenkatalysator eingesetzt. In den Ausführungsbeispielen werden ausschließlich chromhaltige Katalysatoren verwendet. Hohe GBL-Ausbeuten lassen sich bei einem Druck von 2 bar realisieren, wird ein höherer Druck verwandt, steigt die THF-Ausbeute, die GBL-Ausbeute geht zurück.

In der US 5,149,836 wird ein Mehrstufen-Gasphasenverfahren zur Herstellung von GBL und THF mit variablen Produktselektivitäten offenbart, wobei in einer ersten Stufe eine Mischung aus Rein-MSA und Wasserstoff über einen Katalysator geleitet wird, welcher Kupfer, Zink und Aluminium enthält. Dieser rohe Reaktionsaustrag wird anschließend zur Herstellung des THF über einen chromhaltigen Katalysator geführt.

In der WO 99/38856 wird ein lediglich aus Kupfer und Chrom bestehender Katalysator offenbart, mit dem im geraden Durchgang ausgehend von Rein-MSA GBL-Selektivitäten von 92 bis 96 mol-% erhalten werden.

In der EP-A 638 565 wird ein Kupfer-, Chrom- und Silicium-enthaltender Katalysator offenbart, wobei die Zusammensetzung nach einem Beispiel ca. 78% CuO, 20% Cr₂O₃ und 2% SiO₂ entspricht. Mit Rein-MSA und Stickstoff-Wasserstoff-Gemischen konnten GBL-Ausbeute von 98% erhalten werden.

Die nachfolgenden Dokumente offenbaren die Verwendung chromfreier Katalysatoren bei der Hydrierung von MSA.

In der GB-A 1 168 220 ist ein Gasphasen-Verfahren zur Herstellung von GBL offenbart, bei dem MSA oder BSA an einem binären Kupfer-Zink-Katalysator zu GBL hydriert werden. In allen Ausführungsbeispielen wird bei atmosphärischem Druck gearbeitet, wobei ausgehend von Rein-MSA GBL-Ausbeuten von 94 mol-% erreicht werden konnten.

In der deutschen Offenlegungsschrift DE 2 404 493 wird ebenfalls ein Verfahren zur Herstellung von GBL durch katalytische Hydrierung von Gemischen aus MSA, BSA, Maleinsäure (MS), Bernsteinsäure (BS) und Wasser an einem metallischen Katalysator offenbart, neben Kupfer-Chromit-Katalysatoren kommen auch Kupfer-Zink- und Kupfer-Zink-Aluminium-Fällkatalysatoren zur Anwendung.

In der WO 91/16132 ist die Hydrierung von MSA zu GBL offenbart, wobei ein CuO, ZnO und Al₂O₃ enthaltender Katalysator, der bei 150°C bis 350°C reduziert und bei 400 °C aktiviert wird, eingesetzt wird. Durch die Aktivierung soll die Standzeit des Katalysatorsystems erhöht werden.

Ein CuO - ZnO - enthaltender Katalysator wird in US 6297389 offenbart. Mit ihm kann nach Aktivierung Rein-MSA zu GBL umgesetzt werden, wobei im geraden Durchgang ausgehend von Rein-MSA GBL-Ausbeuten von 92 bis 96 % erhalten werden.

Die WO 95/22539 offenbart ein Verfahren zur Herstellung von GBL durch katalytische Hydrierung von MSA und/oder BSA an einem Katalysator der aus Kupfer, Zink und Zirkonium besteht. Hierbei werden ausgehend von Rein-MSA GBL-Ausbeuten von bis zu 99 % erreicht.

In der WO 99/35136 wird ein zweistufiges Verfahren zur Herstellung von GBL und THF offenbart, wobei MSA in einer ersten Stufe mit einem Kupfer-haltigen Katalysator hydriert und dieser Reaktionsaustrag über einen sauren Silizium-Aluminium-haltigen Katalysator geleitet wird.

In der WO 97/24346 wird ein Kupferoxid-Aluminiumoxid-Katalysator beschrieben, mit welchem die Hydrierung von MSA in Ausbeuten von 92 mol-% zu GBL gelingt.

Auch die Umsetzung von GBL zu BDO ist eine seit langem bekannte Reaktion. Die nachfolgend erwähnten Dokumente offenbaren diese Reaktion unter Einsatz chromhaltiger Katalysatoren.

In DE 1 277 233 wird ein Verfahren zur Herstellung von Gemischen verschiedener Alkohole durch Hydrierung von Lactonen mit Wasserstoff offenbart. Als Katalysatoren werden mit Barium versetztes Kupferchromit auf einem inaktiven Aluminiumoxydträger verwendet.

In GB-A 1 230 276 wird ein Verfahren zur Herstellung von BDO aus GBL an einem Kupferoxid-Chromoxid-Katalysator bei einer Temperatur zwischen 180 °C und 230 °C beschrieben.

Nach der DE-A 2 231 986 soll mit Kupfer-Chromit-Katalysatoren, die mit Kalium, Natrium, Rubidium, Aluminium, Titan, Eisen, Kobalt oder Nickel dotiert sind, die Standzeit der Katalysatoren verlängert werden.

Nach der DE-A 2501499 wird zur Herstellung von BDO eine Mischung aus Dioxan, GBL, Wasser und Carbonsäuren eingesetzt. Die beschriebene Reaktion findet bei Hochdruck (170 bar) in der flüssigen Phase, vorzugsweise unter Verwendung des Lösemittels Dioxan statt und es kommen ebenfalls Kupfer-Chromoxid-Katalysatoren zum Einsatz.

Kupferchromitkatalysatoren werden nach der J01121-228-A mit Pd dotiert, um einen höheren Umsatz zu erreichen.

Weitere Kupferchromitkatalysatoren sind beschrieben bei Dasunin, Maeva, Z.Org.chim. 1 (1965), Nr.6, S996-1000; JA 5366/69; JA 7240770; J4 9024-906; J49087-610; wobei in den Ausführungsbeispielen Rein-GBL in der flüssigen Phase zu BDO umgesetzt wird.

Die Gasphasenhydrierung von Rein-GBL zu Butandiol ist in der US 4,652,685 an Kupfer-Chromit-Katalysatoren beschrieben. Hierbei konnte bei einem Druck von 41 bar bei einem Umsatz von 60-68% eine BDO-Selektivität von 92-97 % erreicht werden.

In den US 5,406,004 und US 5,395,990 werden Verfahren zur Herstellung von Gemischen aus Alkoholen und Diolen durch Hydrierung von Rein-GBL an Kupfer-haltigen Katalysatoren offenbart. Hierbei wird eine mit Kupferkatalysator gefüllte Hydrierzone mit Hydrierfeed und Wasserstoff bei einer Temperatur von 150 bis 350°C und Drücken zwischen 10,3 bar und 138 bar beaufschlagt und ein Produkt bestehend aus Alkoholen und Diolen isoliert. In den Ausführungsbeispielen wird eine Reihe von Kupfer, Zink und Chrom enthaltenden Katalysatoren beschrieben.

Schliesslich offenbaren die nachfolgend aufgeführten Dokumente den Einsatz chromfreier Kupferkatalysatoren zur Hydrierung von GBL zu BDO.

Ein aus CuO und ZnO bestehender Katalysator wird in WO 82/03854 beschrieben. Hiermit kann in der Gasphase bei einem Druck von 28,5 bar und einer Temperatur von 217 °C eine BDO-Selektivität von 98,4 % erreicht werden. Der Umsatz an Rein-GBL ist jedoch unbefriedigend gering.

Mit Palladium und Kalium dotierte Kupfer-Tränkkatalysatoren sind in den Dokumenten US 4,797,382; US 4,885,411 und EP-A 0 318 129 beschrieben. Sie sind zur Umsetzung von GBL zu Butandiol geeignet.

Die Verwendung eines Gemisches aus GBL und Wasser als Feedstrom in Verbindung mit einem Kupferoxid-Zinkoxid-Katalysator ist in US 5030773 A beschrieben. Hierin wird offenbart, daß die Aktivität derartiger Katalysatoren zunimmt, falls dem Rein-GBL-Strom 1 bis 6 % Wasser zugemischt werden und dieses Gemisch in der Gasphase hydriert wird. Falls Rein-GBL für diese Reaktion Verwendung findet, so muß extra Wasser zu gemischt werden, welches anschließend wieder abgetrennt werden muß. Falls GBL eingesetzt würde, das durch Hydrierung von MSA entsteht, wären 17% Wasser im Feed enthalten. Demzufolge müssten mindestens 11% Wasser vor der Hydrierung zu BDO abgetrennt werden.

In JP 0 634 567 - A ist ein Kupfer-Eisen-Aluminium-enthaltender Katalysator beschrieben, der sich zur Hydrierung von Rein-GBL zu BDO bei Hochdruck (250 bar) eignet.

Ein Verfahren zur Herstellung von BDO ausgehend von Maleinsäureestern ist in WO 99/35113 aufgeführt. Hierbei wird in drei aufeinanderfolgenden Stufen hydriert. Ausgehend von Maleinsäureester wird an einem edelmetallhaltigen Katalysator Bernsteinsäureester hergestellt, welcher in einer zweiten Stufe zu GBL und THF umgesetzt wird. GBL wird abgetrennt und in einer dritten Stufe bei höherem Druck zu BDO umgesetzt.

In der WO 99/35114 wird ein Verfahren zur Herstellung von BDO durch Flüssigphasenhydrierung von GBL, Bemsteinsäureester oder Gemische der beiden bei Drucken zwischen 60 bar und 100 bar und Temperaturen zwischen 180 °C und 250 °C beschrieben. Als Katalysator wird ein Kupferoxid-Zinkoxid-Katalysator eingesetzt.

Eine weitere Gasphasenvariante der Hydrierung von GBL zu BDO wird in WO 99/52845 an einem Kupferoxid-Zinkoxid-Katalysator offenbart. Zusätzlich zum üblichen Reaktionsfeed wird Kohlenmonoxid dem Wasserstoff beigemischt, um gleichzeitig Methanol herzustellen.

In der EP-A 0 382 050 wird an der Hydrierung von Rein-GBL an einem Cobaltoxid, Kupferoxid, Manganoxid und Molybdänoxid enthaltenden Katalysator gearbeitet.

Auch die direkte Herstellung von BDO ausgehend von MSA ist bekannt. Die nachfolgend aufgeführten Dokumente beschreiben diese Reaktion unter Einsatz von chromhaltigen Katalysatoren.

In der DE 2 845 905 wird ein kontinuierliches Verfahren zur Herstellung von Butandiol ausgehend von Maleinsäureanhydrid beschrieben. Hierbei wird MSA gelöst in einwertigen aliphatischen Alkoholen mit Wasserstoff bei Drücken von 250 bar und 350 bar an Kupferchromitkatalysatoren umgesetzt.

Ein Verfahren zur gleichzeitigen Herstellung von BDO und THF ausgehend von MSA an Kupfer-, Chrom- und Mangan-enthaltenden Katalysatoren ist in der EP-A 0 373 947 offenbart. Es werden Gemische aus MSA und GBL, Gemische aus MSA und 1,4-Dioxan und Rein-MSA eingesetzt. In allen Fällen werden Gemische von THF und BDO erhalten. Nachteilig an diesem Verfahren sind die hohen Ausbeuten an Tetrahydrofuran.

In den Dokumenten CN-A 1 113 831-A, CN-A 1 116 615-A, CN-A 1 138 018-A und CN-A 1 047 328 werden chromhaltige Katalysatoren offenbart. In der CN 1 137 944 A wird ein Kupfer-, Chrom-, Mangan-, Barium-, und Titan-haltiger Katalysator verwendet.

Nach der Offenbarung der CN-A 1 182 639 kann ein Kupfer-, Chrom-, Zink- und Titan-haltiger Katalysator zur Hydrierung von Mischungen aus GBL und MSA benutzt werden.

Die CN-A 1 182 732 beschreibt ein Verfahren zur Herstellung von BDO durch Gasphasenhydrierung von MSA an Kupfer- und Chrom-haltigen Katalysatoren bei 200 bis 250°C und einem Druck von 30 bis 70 bar. Hierbei ist MSA bei der Hydrierung in einem geeigneten Lösemittel gelöst.

Die nachfolgend aufgeführten Dokumente schliesslich offenbaren die direkte Hydrierung von MSA zu BDO unter Einsatz chromfreier Katalysatoren.

So wird in der DE-A 2 455 617 ein dreistufiges Verfahren zur Herstellung von BDO beschrieben. In einer ersten Stufe werden an einem Ni-haltigen Katalysator Lösungen von MSA in GBL zu BSA in GBL hydriert. In einer zweiten Stufe bei hohem Druck (80-200 bar) und höherer Temperatur wird diese Lösung aus BSA in GBL in der flüssigen Phase zu GBL hydriert, anschließend Wasser, Bernsteinsäureanhydrid und Bernsteinsäure vom GBL abgetrennt und das reine GBL teilweise zurückführt und in einer dritten Verfahrensstufe an einem Kupfer-Zinkoxid-Katalysator in der flüssigen Phase bei hohem Druck zu Butandiol umgesetzt.

In der US 4,301,077 wird ein Ruthenium-haltiger Katalysator zur Hydrierung von MSA zu BDO eingesetzt.

Die DE-A 3 726 510 offenbart die Verwendung eines Kupfer, Kobalt und Phosphor enthaltenden Katalysators zur direkten Hydrierung von MSA.

Ein reiner Kupferoxid-Zinkoxid-Katalysator kommt bei J0 2025-434-A zum Einsatz. Laut den Ausführungsbeispielen kann Rein-MSA bei einem Druck von 40 bar umgesetzt werden. Die Ausbeute an Butandiol beträgt jedoch lediglich 53,5 mol-%, als Nebenausbeute werden 40,2 mol-% GBL gefunden.

In der EP-A 373 946 wird ein Verfahren offenbart, bei dem man einem mit Rhenium dotierten Kupferoxid-Zinkoxid-Katalysator MSA der Gasphase direkt zu BDO umgesetzt wird.

Die gleichzeitige Herstellung von BDO und THF ist Gegenstand der Patentanmeldungen J0 2233-627-A (hier wird ein Kupfer-Zink-Aluminium-Katalysator eingesetzt) J0 2233-630-A (hier wird ein Mangan-, Barium- und Silicium-enthaltender Kupfer-Chrom-Katalysator verwendet) und J0 2233-631-A (hier wird ein Kupfer und Aluminium enthaltender Katalysator eingesetzt). Durch Anwendung dieser Katalysatoren werden neben BDO aus MSA neben BDO auch große Mengen THF gebildet.

Ein Katalysator enthaltend Kupfer-, Mangan- und Kalium ist in der J0-A 2233-632 beschrieben.

In EP-A 431 923 wird ein zweistufiges Verfahren zur Herstellung von BDO und THF beschrieben, wobei in einer ersten Stufe GBL durch Flüssigphasen-Hydrierung von MSA entsteht und dieses in einer zweiten Stufe durch Gasphasenreaktion an einem Kupfer-Silizium-enthaltenden Katalysator zu Butandiol umgesetzt wird.

Die US 5,196,602 offenbart ein Verfahren zur Herstellung von Butandiol durch Hydrierung von MSA oder Maleinsäure mit Wasserstoff in einem zweistufigen Prozeß. In einer ersten Stufe wird MSA zu BSA und / oder GBL hydriert, welches in einer zweiten Stufe in Gegenwart eines Ru-enthaltenden Katalysators zu BDO umgesetzt wird.

Die den oben zitierten Druckschriften zugrundeliegenden Technologien benutzen als Edukt für die Hydrierungsreaktionen vorgereinigtes MSA, das nach seiner Herstellung im allgemeinen durch Destillation von Verunreinigungen befreit wurde. MSA wird hergestellt durch partielle Oxidation von bestimmten Kohlenwasserstoffen, nämlich Benzol, Butengemischen sowie n-Butan, wobei dieses letztere vorzugsweise eingesetzt wird. Das Rohprodukt der Oxidation enthält neben dem gewünschten MSA vor allem Nebenprodukte wie Wasser, Kohlenmonoxid, Kohlendioxid, nicht umgesetzten Ausgangskohlenwasserstoff sowie Essig- und Acrylsäure, wobei diese Nebenprodukte unabhängig von den in die Oxidation eingesetzten Kohlenwasserstoffen sind. Zumeist werden die Nebenprodukte durch aufwendige Verfahren abgetrennt, beispielsweise durch Destillation, wie oben erwähnt. Das Reinigen erweist sich insbesondere als notwendig, weil die in den Hydrierverfahren eingesetzten Katalysatoren generell empfindlich auf solche Verunreinigungen reagieren. Die Desaktivierung der Katalysatoren ist bereits bei Einsatz von gereinigtem MSA ein Problem, da durch Belegung mit dessen Polymerisationsprodukten der Katalysator in der Regel in relativ kurzen Intervallen, die oftmals bei ca. 100 Stunden liegen, regeneriert werden muß. Die Tendenz zur Desaktivierung ist beim Vorliegen polymerisierbarer Verunreinigungen, wie beispielsweise der Acrylsäure, noch erhöht. Diese Tatsache ist dem Fachmann bekannt und wird beispielsweise auch in den Patentanmeldungen EP-A 322 140, WO 91/16132 und DE-OS 240 44 93 beschrieben.

Bis jetzt existiert im Stand der Technik lediglich eine Druckschrift, die die Hydrierung von lediglich grob vorgereinigtem MSA offenbart. Aus der WO 97/43234 ist bekannt, Maleinsäureanhydrid aus Maleinsäureanhydrid enthaltenden Gasströmen, die aus der Oxidation von Kohlenwasserstoffen stammen, mit Hilfe von mindestens 30 °C höher siedenden Absorptionsmitteln zu absorbieren, das Maleinsäureanhydrid aus diesen Absorptionsmitteln mit Hilfe von Wasserstoff herauszutreiben und den Maleinsäureanhydrid enthaltenden Wasserstoffstrom in der Gasphase an einem heterogenen Katalysator zu hydrieren. Dabei wird hauptsächlich BDO neben geringen Mengen GBL und THF erhalten. Die Hydrierung wird bei etwa 150 □C bis 300 □C und einem Druck von 5 bar bis 100 bar in der Gasphase durchgeführt. Als Katalysatoren werden promotierte Kupferkatalysatoren verwendet, wie sie in Journal of Catalysis 150, Seiten 177 bis 185 (1994) beschrieben sind. Dabei handelt es sich um chromhaltige Katalysatoren des Typs Cu/Mn/Ba/Cr und Cu/Zn/Mg/Cr. Somit werden gemäß der Offenbarung dieser Anmeldung chromhaltige Katalysatoren zur Hydrierung von MSA-Qualitäten, die die oben dargelegten Verunreinigungen aufweisen, eingesetzt. Der Einsatz chromhaltiger Katalysatoren wird jedoch aufgrund der Toxizität heutzutage so weit wie möglich vermieden.

Neuere Technologien rücken aufgrund der Toxizität mehr und mehr von der Verwendung chromhaltiger Katalysatoren ab. Beispiele für chromfreie Katalysatorsysteme finden sich in den Druckschriften WO 99/35139 (Cu-Zn-Oxid), WO 95/22539 (Cu-Zn-Zr) sowie der US 5,122,495 (Cu-Zn-Al-Oxid).

Auf dem Gebiet der Hydrierung von MSA zu Folgeprodukten, insbesondere GBL, THF und/oder BDO, existiert also ein nahezu unüberschaubarer Stand der Technik, wobei vorgehend nur eine Auswahl aus dem gesamten existierenden Stand der Technik zitiert wurde.

Zusammenfassend läßt sich sagen, daß die bei der Herstellung von BDO durch Hydrierung von MSA auftretenden technischen Probleme gelöst wurden dahingehend, daß befriedigende Ausbeuten und Selektivitäten zu BDO- also nur untergeordnete Bildung von THF - erzielt werden. Dies wurde erreicht durch unterschiedliche Massnahmen bzw. die Kombination verschiedener Massnahmen.

Generell wurde BDO durch direkte Hydrierung von Rein-GBL - das wiederum durch Hydrierung von MSA und anschliessende aufwendige Reinigung gewonnen wurde - erhalten. In jedem Fall wurde als Edukt Rein-MSA eingesetzt, das nur noch geringe Mengen an Verunreinigungen enthielt, da ansonsten keine zufriedenstellende Selektivität und Katalysatorstandzeit erreicht wurden. Es wurden chromhaltige Katalysatoren eingesetzt, insbesondere auch in der zweiten Stufe, um eine hohe BDO-Selektivität und die gewünschte Standzeit zu erreichen. Zur Vermeidung des Einsatzes chromhaltiger Katalysatoren konnte alternativ auf edelmetallhaltige Katalysatoren zurückgegriffen werden, die bezüglich Ausbeute, Selektivität und auch Standfestigkeit mit chromhaltigen Katalysatoren vergleichbar, jedoch deutlich kostenintensiver sind.

Weiterhin wurde bei der bevorzugten Durchführung der Reaktion in zwei getrennten Stufen nach der ersten Hydrierstufe eine aufwendige Reinigung des GBL durchgeführt, um eine hohe Standzeit der Katalysatoren, insbesondere auch im Hinblick auf die gewünschte Selektivität, zu erreichen. Bis jetzt ist lediglich in der erwähnten WO 97/43234 ein Verfahren offenbart, bei dem MSA, das nur grob vorgereinigt wurde, als Edukt zur Herstellung von BDO durch Hydrierung eingesetzt wird. Das Verfahren wird einstufig durchgeführt, vermeidet also auch die Aufarbeitung nach der ersten Hydrierstufe. Jedoch eignen sich lediglich chromhaltige Katalysatoren für diese Umsetzung.

Gewünscht wird ist ein Verfahren zur Herstellung von BDO aus MSA, das mit möglichst geringem apparativen Aufwand die Gewinnung von BDO in guten Ausbeuten ermöglicht. Weiterhin ist erwünscht, dass in dem Verfahren kein Rein-MSA eingesetzt werden muss und keine Zwischenreinigung der Reaktionsprodukte der ersten Stufe notwendig ist. Weiterhin soll das Verfahren keine chromhaltigen Katalysatoren benötigen, vorzugsweise auch keine Katalysatoren, die Edelmetalle enthalten sowie eine hohe Selektivität zu BDO aufweisen, insbesondere wenig THF liefern.

Diese Aufgabe wird gelöst durch ein Verfahren zur Herstellung von gegebenenfalls alkylsubstituiertem 1,4-Butandiol durch zweistufige katalytische Hydrierung in der Gasphase von C₄-Dicarbonsäuren und/oder deren Derivaten mit den folgenden Schritten:
a) Einführen eines Gasstroms einer C₄-Dicarbonsäure oder eines Derivates davon bei 200 bis 300°C und 10 bis 100 bar in einen ersten Reaktor oder in eine erste Reaktionszone eines Reaktors und katalytische Gasphasenhydrierung zu einem hauptsächlich, gegebenenfalls alkylsubstituiertes γ-Butyrolacton enthaltenden Produkt;
b) Einführen des so erhaltenen Produktstroms in einen zweiten Reaktor oder in eine zweite Reaktionszone eines Reaktors bei einer Temperatur von 140°C bis 260°C und katalytische Gasphasenhydrierung zu gegebenenfalls alkylsubstituiertem 1,4-Butandiol,
   wobei die Schritte a) und b) bei gleichem Druck durchgeführt werden;
c) Abtrennen des gewünschten Produkts von Zwischenprodukten, Nebenprodukten und gegebenenfalls nicht umgesetztem Edukt;
d) gegebenenfalls Rückführen von nicht umgesetzter Zwischenprodukten in eine oder beide Hydrierstufen,
   wobei in beiden Hydrierstufen jeweils ein Katalysator verwendet wird, der ≤ 95 Gew.-%, vorzugsweise 5 bis 95 Gew.-%, insbesondere 10 bis 80 Gew.-% CuO und ≥ 5 Gew.-%, vorzugsweise 5 bis 95 Gew.-%, insbesondere 20 bis 90 Gew.-% eines oxidischen Trägers aufweist, und das der ersten Hydrierstufe entnommene Produktgemisch ohne weitere Reinigung in die zweite Hydrierstufe eingeführt wird.

Dabei wird unter "gleicher Druck" in den Schritten a) und b) im Sinne der vorliegenden Anmeldung verstanden, dass der den ersten Reaktor oder die erste Reaktionszone verlassende Produktstrom weder verdichtet noch entspannt wird, bevor dieser in den zweiten Reaktor oder in die zweite Reaktionszone eintritt. Die Reaktion wird im allgemeinen in einem Hydrierkreis, unter Verwendung lediglich eines Verdichters, durchgeführt. Gewisse Druckschwankungen zwischen dem ersten und dem zweiten Reaktor bzw. der ersten und der zweiten Reaktionszone können durch dem Fachmann bekannte Gegebenheiten auftreten, die generell apparativ bedingt sind. Die Druckschwankungen können dabei bis zu wenigen bar betragen.

Mit Hilfe des erfindungsgemäßen Verfahrens wird der apparative Aufwand gegenüber den aus dem Stand der Technik bekannten zweistufigen Verfahren, bei denen die zweite Stufe (Reduktion von GBL zu BDO) bei höheren Drücken durchgeführt wird als die erste Stufe (Reduktion von MSA zu GBL), wesentlich verringert. Es ergibt sich eine kostengünstige Verfahrensdurchführung.

Das erfindungsgemäße Verfahren ermöglicht das Erzielen hoher BDO-Selektivitäten.

Um die gewünschten BDO-Selektivitäten erzielen zu können, ist das Einhalten gewisser Reaktionsparameter in beiden Hydrierstufen erforderlich, diese Parameter werden nachfolgend aufgeführt.

Nachfolgend soll im Sinne der vorliegenden Anmeldung unter "erstem Reaktor" sowohl "erster Reaktor" als auch - wenn die erste und zweite Hydrierstufe in einem Reaktor mit mehreren Reaktionszonen durchgeführt werden - "erste Reaktionszone" verstanden werden. Ebenso soll unter "zweitem Reaktor" sowohl "zweiter Reaktor" als auch - wenn die erste und zweite Hydrierstufe in einem Reaktor mit mehreren Reaktionszonen durchgeführt werden - "zweite Reaktionszone" verstanden werden.

In dem erfindungsgemässen Verfahren können Edukte unterschiedlicher Reinheit in der Hydrierreaktion eingesetzt werden. Selbstverständlich kann ein Edukt hoher Reinheit, insbesondere MSA, in der Hydrierreaktion eingesetzt werden. Ein Vorteil des erfindungsgemässen Verfahrens liegt aber darin, dass auch der Einsatz von Edukten, insbesondere MSA, das mit den üblichen, bei der Oxidation von Benzol, Butenen oder n-Butan anfallenden Verbindungen sowie eventuell weiteren Komponenten verunreinigt ist, ermöglicht wird. Somit kann das erfindungsgemässe Hydrierverfahren in einer weiteren Ausführungsform eine vorgeschaltete Stufe umfassen, die das Herstellen des zu hydrierenden Edukts durch partielle Oxidation eines geeigneten Kohlenwasserstoffs sowie das Abtrennen des zu hydrierenden Edukts aus dem damit erhaltenen Produktstrom umfaßt. Dabei wird vorzugsweise nur ein grobes Abtrennen durchgeführt, das keinen unnötig hohen Aufwand erfordert und bei dem noch eine in den bislang bekannten Verfahren nicht tolerierbare Menge an Verunreinigungen in dem Edukt verbleibt.

Insbesondere ist dieses zu hydrierende Edukt MSA. Dabei wird bevorzugt MSA eingesetzt, welches aus der Partialoxidation von Kohlenwasserstoffen stammt. Geeignete Kohlenwasserstoffströme sind Benzol, C₄-Olefine (z.B. n-Butene, C₄-Raffinatströme) oder n-Butan. Besonders bevorzugt eingesetzt wird n-Butan, da es einen preiswerten, wirtschaftlichen Einsatzstoff darstellt. Verfahren zur Partialoxidation von n-Butan sind beispielsweise in Ullmann's Encyclopedia of Industrial Chemistry, 6^{th} Edition, Electronic Release, Maleic and Fumaric Acids - Maleic Anhydride beschrieben.

Der so erhaltene Reaktionsaustrag wird dann vorzugsweise in einem geeigneten organischen Lösungsmittel oder -Gemisch aufgenommen, das bei Atmosphärendruck einen um mindestens 30°C höheren Siedepunkt als MSA hat.

Dieses Lösungsmittel (Absorptionsmittel) wird auf eine Temperatur im Bereich zwischen 20 und 160 °C, bevorzugt zwischen 30 und 80 °C, gebracht. Der Maleinsäureanhydrid enthaltende Gasstrom aus der Partialoxidation kann in vielfältiger Weise mit dem Lösungsmittel in Kontakt gebracht werden: (i) Einleiten des Gasstroms in das Lösungsmittel (z.B. über Gaseinleitungsdüsen oder Begasungsringe), (ii) Einsprühen des Lösungsmittels in den Gasstrom und (iii) Gegenstromkontakt zwischen dem nach oben strömenden Gasstrom und dem nach unten strömenden Lösungsmittel in einer Boden- oder Packungskolonne. In allen drei Varianten können die dem Fachmann bekannten Apparate zur Gasabsorption eingesetzt werden. Bei der Wahl des einzusetzenden Lösungsmittels ist darauf zu achten, daß dies nicht mit dem Edukt, beispielsweise dem vorzugsweise eingesetzten MSA, reagiert. Geeignete Lösungsmittel sind: Trikresylphosphat, Dibutylmaleat, Butylinaleat, hochmolekulare Wachse, aromatische Kohlenwasserstoffe mit einem Molekulargewicht zwischen 150 und 400 und einem Siedepunkt oberhalb 140 °C, wie beispielsweise Dibenzylbenzol; Alkylphthalate und Dialkylphthalate mit C₁-C₁₈-Alkylgruppen, beispielsweise Dimethylphthalat, Diethylphthalat, Dibutylphthalat, Di-n-Propyl-und Di-iso-Propylphthalat, Undecylphthalat, Diundecylphthalat, Methylphthalat, Ethylphthalat, Butylphthalat, n-Propyl- und iso-Propylphthalat; Di-C₁-C₄-Alkylester anderer aromatischer und aliphatischer Dicarbonsäuren, beispielsweise Dimethyl-2,3-Naphthalin-Dicarbonsäure, Dimethyl-1,4-Cyclohexan-Dicarbonsäure, C₁-C₄-Alkylester anderer aromatischer und aliphatischer Dicarbonsäuren, beispielsweise Methyl-2,3-Naphthalin-Dicarbonsäure, Methyl-1,4-Cyclohexan-Dicarbonsäure, Methylester langkettiger Fettsäuren mit beispielsweise 14 bis 30 Kohlenstoffatomen, hochsiedende Ether, beispielsweise Dimethylether von Polyethylenglykol, beispielsweise Tetraethylenglykoldimethylether.

Der Einsatz von Phthalaten ist bevorzugt.

Die nach der Behandlung mit dem Absorptionsmittel resultierende Lösung hat generell einen MSA-Gehalt von etwa 5 bis 400 Gramm pro Liter.

Der nach der Behandlung mit dem Absorptionsmittel verbleibende Abgasstrom enthält hauptsächlich die Nebenprodukte der vorangegangenen Partialoxidation, wie Wasser, Kohlenmonoxid, Kohlendioxid, nicht umgesetzte Butane, Essig- und Acrylsäure. Der Abgasstrom ist praktisch frei von MSA.

Anschliessend wird das gelöste MSA aus dem Absorptionsmittel ausgetrieben. Dies erfolgt mit Wasserstoff bei oder maximal 10% oberhalb des Druckes der anschließenden Hydrierung oder alternativ im Vakuum mit anschließender Kondensation von verbleibendem MSA. In der Strippkolonne wird ein Temperaturprofil beobachtet, das sich aus den Siedepunkten von MSA am Kopf und dem nahezu MSA-freien Absorptionsmittel am Sumpf der Kolonne bei dem jeweiligen Kolonnendruck und der eingestellten Verdünnung mit Trägergas (im ersten Fall mit Wasserstoff) ergibt.

Um Verluste an Lösungsmittel zu verhindern, können sich oberhalb der Zufuhr des Roh-MSA-Stromes Rektifiziereinbauten befinden. Das vom Sumpf abgezogene, nahezu MSAfreie Absorptionsmittel wird wieder der Absorptionszone zugeführt. Das H₂/MSA-Verhältnis liegt bei etwa 20 bis 600. Im anderen Fall wird das kondensierte MSA in einen Verdampfer gepumpt und dort in den Kreisgasstrom verdampft.

Der MSA-Wasserstoff-Strom enthält noch Nebenprodukte, die bei der partiellen Oxidation von n-Butan, Butenen oder Benzol mit Sauerstoff enthaltenden Gasen entstehen, sowie nicht abgetrenntes Absorptionsmittel. Hierbei handelt es sich vor allem um Essigsäure und Acrylsäure als Nebenprodukte, Wasser, Maleinsäure sowie die vorzugsweise als Absorptionsmittel verwendeten Dialkylphthalate. Das MSA enthält Essigsäure in Mengen von 0,01 bis 1 Gew.-%, vorzugsweise 0, 1 bis 0,8 Gew.-% und Acrylsäure in Mengen von 0,01 bis 1 Gew.-%, vorzugsweise 0,1 bis 0,8 Gew.-%, bezogen auf MSA. In der Hydrierstufe werden Essigsäure und Acrylsäure ganz oder teilweise zu Ethanol bzw. Propanol hydriert. Der Maleinsäure-Gehalt beträgt 0,01 bis 1 Gew.-%, insbesondere 0,05 bis 0,3 Gew.-%, bezogen auf MSA.

Werden Dialkylphthalate als Absorptionsmittel eingesetzt, hängt deren Gehalt im MSA stark vom richtigen Betrieb der Strippkolonne, insbesondere vom Verstärkungsteil ab. Phthalatgehalte von bis 1,0 Gew.-% , insbesondere bis 0,5 Gew.-% sollten bei geeigneter Betriebsweise nicht überschritten werden, da sonst der Verbrauch an Absorptionsmittel zu hoch wird.

Der vorzugsweise wie vorstehend beschrieben erhaltene Wasserstoff/Maleinsäureanhydrid-Strom wird nun dem ersten Hydrierreaktor oder der ersten Hydrierzone eines Reaktors zugeführt und hydriert. Die Katalysatoraktivitäten und -standzeiten sind dabei verglichen mit dem Einsatz von stark, beispielsweise durch Destillation, vorgereinigtem MSA praktisch unverändert.

Der aus dem ersten Reaktor oder der ersten Hydrierzone eines Reaktors austretende Gasstrom wird mit gegebenenfalls rückgeführtem GBL der zweiten Hydrierung zugeführt.

Das Rückführgas der zweiten Stufe kann in den Eingang der ersten Stufe rückgeführt werden.

Bei allen Reaktionsvarianten kann der aus dem zweiten Reaktor austretende Gasstrom, vorzugsweise auf 10 bis 60°C gekühlt. Dabei werden die Reaktionsprodukte auskondensiert und in einen Abscheider geleitet. Der nichtkondensierte Gasstrom kann vom Abscheider abgezogen und dem Kreisgasverdichter zugeführt. Entstandene Nebenprodukte im rückgeführten Kreisgasstrom können durch dem Fachmann bekannte Massnahmen entfernt werden, vorzugsweise durch Ausschleusen einer kleinen Menge an Kreisgas. Die auskondensierten Reaktionsprodukte werden dem System entnommen und einer Aufarbeitung zugeführt. Als Koppel- und Nebenprodukte findet man in der auskondensierten Flüssigkeitsphase hauptsächlich THF, n-Butanol neben geringen Mengen an Propanol.

Aus dem flüssigen Hydrieraustrag der zweiten Stufe werden dann die Nebenprodukte sowie Wasser abgetrennt und das gewünschte Produkt BDO isoliert. Dies geschieht generell durch fraktionierende Destillation. Neben- und Zwischenprodukte wie beispielsweise GBL und Di-BDO können in die Hydrierung der ersten und/oder zweiten Stufe, vorzugsweise der zweiten Stufe zurückgeführt oder alternativ destillativ aufgearbeitet werden.

Das erfindungsgemäße Verfahren kann diskontinuierlich, semi-kontinuierlich oder kontinuierlich durchgeführt werden. Die kontinuierliche Durchführung ist bevorzugt.

Ein wichtiger Parameter ist das Einhalten einer geeigneten Reaktionstemperatur in beiden Hydrierstufen.

In der ersten Hydrierstufe wird dies vorzugsweise erreicht durch eine genügend hohe Temperatur der Edukte beim Eintritt in den ersten Hydrierreaktor oder in die erste Reaktionszone eines Reaktors. Diese sogenannte Anfangshydriertemperatur liegt bei Werten von 200 bis 300°C, vorzugsweise 235 bis 270°C. Um die gewünschte Selektivität und Ausbeute in der ersten Stufe zu erhalten, soll die Reaktion weiterhin vorzugsweise so durchgeführt werden, daß am Katalysatorbett, an dem die eigentliche Reaktion stattfindet, eine geeignet hohe Reaktionstemperatur herrscht. Diese sogenannte Hot-Spot-Temperatur wird nach dem Eintritt der Edukte in den Reaktor eingestellt und liegt vorzugsweise bei Werten von 210 bis 310°C, insbesondere 245 bis 280°C. Das Verfahren wird vorzugsweise so durchgeführt, daß die Eingangstemperatur und die Austrittstemperatur der Reaktionsgase unterhalb dieser Hot-Spot-Temperatur liegen. Die Hot-Spot-Temperatur liegt dabei vorteilhafterweise in der 1. Hälfte des Reaktors, insbesondere bei Vorliegen eines Rohrbündelreaktors. Vorzugsweise liegt die Hot-Spot-Temperatur 5 bis 30°C, insbesondere 5 bis 15°C, besonders bevorzugt bei 5 bis 10°C, oberhalb der Eintrittstemperatur. Wenn die Hydrierung unterhalb der Minimaltemperaturen der Eingangs- bzw. Hot-Spot-Temperatur durchgeführt wird, dann steigt generell im Fall der Verwendung von MSA als Edukt die Menge an BSA bei gleichzeitiger Verminderung der Menge an GBL und BDO an. Weiterhin sind bei einer solchen Temperatur im Verlauf der Hydrierung eine Desaktivierung des Katalysators durch Belegung mit Bernsteinsäure, Fumarsäure und/oder BSA und eine mechanische Schädigung des Katalysators zu beobachten. Wird dagegen mit MSA als Edukt oberhalb der Maximaltemperaturen der Eingangs- bzw. Hot-Spot-Temperatur hydriert, sinken die BDO-Ausbeute und Selektivität generell auf nicht zufriedenstellende Werte. Es ist hierbei die vermehrte Bildung von THF, n-Butanol und n-Butan zu beobachten, also den Produkten einer weiteren Hydrierung.

Die Eingangstemperatur (Anfangshydriertemperatur) in den zweiten Reaktor ist im allgemeinen gleich der Eingangstemperatur in den ersten Reaktor oder niedriger, wobei eine niedrigere Eingangstemperatur in den zweiten Reaktor gegenüber der Eingangstemperatur in den ersten Reaktor bevorzugt ist.

In der zweiten Hydrierstufe liegt die Eingangstemperatur (Anfangshydriertemperatur) bei Werten von 140°C bis 260°C, vorzugsweise 160°C bis 225°C, insbesondere bei 180 bis 200°C. Wenn die Hydrierung unterhalb der Minimaltemperaturen der Eingangstemperatur durchgeführt wird, sinkt die Menge an gebildetem BDO. Der Katalysator verliert an Aktivität. Weiterhin ist unterhalb der Minimaltemperatur mit einem Auskondensieren der Einsatzstoffe und einer Schädigung des Kupferkatalysators durch Wasser zu rechnen. Wird dagegen mit GBL als Edukt oberhalb der Maximaltemperaturen der Eingangs-Temperatur hydriert, sinken die BDO-Ausbeute und Selektivität auf nicht zufriedenstellende Werte. Bei diesen Temperaturen liegt das Hydriergleichgewicht zwischen BDO und GBL auf Seiten des GBL, so dass weniger Umsatz erzielt wird, jedoch ist die Nebenproduktbildung durch Überhydrierung zu THF, n-Butanol und n-Butan bei höherer Temperatur verstärkt zu beobachten.

Die Anfangshydriertemperatur kann in beiden Hydrierstufen die gleiche sein. Vorzugsweise ist die Anfangshydriertemperatur in der zweiten Hydrierstufe niedriger als in der ersten Hydrierstufe.

Die Temperaturerhöhung des Gasstromes im Reaktor sollte 110°C, vorzugsweise 40°C nicht überschreiten, insbesondere nicht mehr als 20°C sein. Auch hier führen grosse Temperaturerhöhungen häufig zu Überhydrierreaktionen und einem (BDO+GBL) Selektivitätsverlust.

Der Druck beträgt sowohl in der ersten Hydrierstufe als auch in der zweiten Hydrierstufe 10 bis 100 bar, bevorzugt 15 bis 50 bar, besonders bevorzugt wird ein Druck von 20 bis 40 bar gewählt. In diesem Druckbereich ist bei der Hydrierung von MSA die Bildung von THF aus dem zunächst entstehenden Zwischenprodukt GBL weitestgehend unterdrückt. Bei den in der zweiten Hydrierstufe angelegten Temperaturen ist der Umsatz an GBL zu BDO hoch, die THF-Bildung gering. Insbesondere bei Anlegen einer niedrigen Hydriertemperatur in der zweiten Hydrierstufe gegenüber der ersten Hydrierstufe sind diese Effekte deutlich.

Die Katalysatorbelastung der ersten Hydrierstufe liegt vorzugsweise im Bereich von 0,02 bis 1, insbesondere 0,05 bis 0,5 kg Edukt/1 Katalysator • Stunde. Wird die Katalysatorbelastung der ersten Stufe im Falle von MSA über den genannten Bereich hinaus erhöht, ist generell eine Erhöhung des Anteils an BSA und Bernsteinsäure im Hydrieraustrag zu beobachten. Vorzugsweise liegt die Katalysatorbelastung der zweiten Hydrierstufe im Bereich von 0,02 bis 1,5, insbesondere 0,1 bis 1 kg Edukt/1 Katalysator • Stunde. Wird die Katalysatorbelastung über den genannten Bereich hinaus gesteigert, ist mit unvollständigem Umsatz an GBL zu rechnen. Dies kann gegebenenfalls durch eine erhöhte Rückführrate ausgeglichen werden, dies ist jedoch selbstverständlich nicht bevorzugt.

Weiterhin ist das Wasserstoff/Edukt-Molverhältnis ein Parameter, der einen Einfluss auf die Produktverteilung und auch die Wirtschaftlichkeit des erfindungsgemässen Verfahrens hat. Aus wirtschaftlicher Sicht ist ein niedriges Wasserstoff/Edukt-Verhältnis wünschenswert. Die Untergrenze liegt bei einem Wert von 5, wobei jedoch generell höhere Wasserstoff/Edukt-Molverhältnisse von 20 bis 600, vorzugsweise 40 bis 400, insbesondere 60 bis 350, angewendet werden.

Um die erfindungsgemäss verwendeten Wasserstoff/Edukt-Molverhältnisse einstellen, wird üblicherweise ein Teil, vorteilhafterweise die Hauptmenge, des Wasserstoffs sowohl in der ersten als auch in der zweiten Hydrierstufe im Kreis gefahren. Hierzu setzt man im allgemeinen die dem Fachmann bekannten Kreisgasverdichter ein. Ein Vorteil der vorliegenden Erfindung liegt darin, dass lediglich ein Hydrierkreis aufgebaut werden muss und die Bereitstellung lediglich eines Verdichters notwendig ist, bei Durchführung von zwei Hydrierstufen.

Die chemisch durch die Hydrierung verbrauchte Wasserstoffmenge wird ergänzt. In einer bevorzugten Ausführungsform wird ein Teil des Kreisgases ausgeschleust, um Inertverbindungen, beispielsweise n-Butan, zu entfernen. Der im Kreis geführte Wasserstoff kann auch, gegebenenfalls nach Vorheizen, zum Verdampfen des Eduktstroms benutzt werden.

Gemeinsam mit dem Wasserstoff-Kreisgas werden alle Produkte im Kreis geführt, die beim Kühlen der aus den Hydrierreaktoren austretenden Gasströme nicht oder nicht vollständig auskondensieren. Dies sind vor allem THF, Wasser und Nebenprodukte wie Methan und Butan. Die Kühltemperatur beträgt vorzugsweise 0 bis 60°C, vorzugsweise 20 bis 45°C.

Als Reaktortypen kommen alle, für heterogen katalysierte Reaktionen mit einem gasförmigen Edukt- und Produktstrom geeigneten Apparate in Betracht. Bevorzugt sind Rohrreaktoren, Schachtreaktoren oder Reaktoren mit innerer Wärmeabfuhr, beispielsweise Rohrbündelreaktoren, es ist auch der Einsatz eines Wirbelbetts möglich. Besonders bevorzugt eingesetzt werden für die erste Hydrierstufe Rohrbündelreaktoren, für die zweite Hydrierstufe werden Schachtreaktoren besonders bevorzugt. Es können sowohl in der ersten als auch der zweiten Hydrierstufe mehrere Reaktoren parallel oder hintereinander geschaltet eingesetzt werden. Prinzipiell kann zwischen die Katalysatorbetten eine Zwischeneinspeisung erfolgen. Möglich ist auch eine Zwischenkühlung zwischen oder in den Katalysatorbetten. Bei Einsatz von Festbettreaktoren ist eine Verdünnung des Katalysators durch Inertmaterial möglich. Das erfindungsgemäße Verfahren wird ganz besonders bevorzugt in einem Zweizonenreaktor durchgeführt.

Ein wichtiger Punkt der vorliegenden Erfindung ist die Wahl der Katalysatoren für beide Stufen, die als katalytisch aktiven Hauptbestandteil Kupferoxid aufweisen. Dieses ist auf einem oxidischen Träger, der eine geringe Anzahl an sauren Zentren besitzen darf, angebracht. Wird ein Katalysator mit einer zu hohen Anzahl an sauren Zentren verwendet, wird BDO dehydratisiert und es entsteht THF.

Ein geeignetes Trägermaterial, das eine ausreichend geringe Anzahl an sauren Zentren besitzt, ist ein Material ausgewählt aus der Gruppe ZnO, Al₂O₃, SiO₂, TiO₂, ZrO₂, CeO₂, MgO, CaO, SrO, BaO und Mn₂O₃ und Mischungen davon. Bevorzugt sind als Trägermaterialien ZnO-/Al₂O₃-Gemische, die delta-, theta-, alpha- und eta-Modifikationen des Al₂O₃ sowie Mischungen, die mindestens eine Komponente aus der Gruppe SiO₂, TiO₂, ZrO₂ einerseits und aus der Gruppe ZnO, MgO, CaO, SrO und BaO andererseits enthalten. Besonders bevorzugt als Trägermaterialien sind reines ZnO, ZnO/Al₂O₃-Gemische im Gewichtsverhältnis 100:1 bis 1:2, sowie Mischungen von SiO₂ mit MgO, CaO und/oder ZnO im Gewichtsverhältnis 200:1 bis 1:1.

Die Menge an Kupferoxid liegt bei Werten ≤ 95 Gew.-%, vorzugsweise 5 bis 95 Gew.-%, insbesondere bei 15 bis 80 Gew.-%; der Träger wird in Mengen von ≥ 5 Gew.-%, vorzugsweise 5 bis 95 Gew.-%, insbesondere 20 bis 85 Gew.-% eingesetzt.

Aufgrund der Toxizität chromhaltiger Katalysatoren werden bevorzugt chromfreie Katalysatoren eingesetzt. Selbstverständlich eignen sich technisch auch entsprechende, dem Fachmann bekannte chromhaltige Katalysatoren für einen Einsatz in dem erfindungsgemässen Verfahren, wodurch sich jedoch nicht die gewünschten Vorteile, die insbesondere umwelt- und arbeitstechnischer Natur sind, ergeben.

In beiden Hydrierstufen kann der gleiche Katalysator verwendet werden, bevorzugt ist jedoch der Einsatz unterschiedlicher Katalysatoren.

Optionsweise können die erfindungsgemäss verwendeten Katalysatoren, ein oder mehrere weitere Metalle oder eine Verbindung davon, vorzugsweise ein Oxid, aus den Gruppen 1 bis 14 (IA bis VIIIA und IB bis IVB der alten IUPAC-Nomenclatur) des Periodensystems der Elemente enthalten. Wird ein weiteres Metall verwendet, wird vorzugsweise Pd eingesetzt in Mengen von ≤ 1 Gew.-%, vorzugsweise ≤ 0,5 Gew.-%, insbesondere ≤ 0,2 Gew.-%. Die Zugabe eines weiteren Metalls oder Metalloxids ist jedoch nicht bevorzugt.

Die eingesetzten Katalysatoren können zudem ein Hilfsmittel in einer Menge von 0 bis 10 Gew.-% enthalten. Unter Hilfsmittel versteht man organische und anorganische Stoffe, die zu einer verbesserten Verarbeitung während der Katalysatorherstellung und/oder zu einer Erhöhung der mechanischen Festigkeit der Katalysatorformkörper beitragen. Derartige Hilfsmittel sind dem Fachmann bekannt; Beispiele umfassen Graphit, Stearinsäure, Kieselgel und Kupferpulver.

Die Katalysatoren lassen sich nach dem Fachmann bekannten Methoden herstellen. Bevorzugt sind Verfahren, bei denen das Kupferoxid fein verteilt und innig vermischt mit den anderen Bestandteilen anfällt, besonders bevorzugt sind Auftränken und Fällungsreaktionen.

Diese Ausgangsmaterialien können nach bekannten Methoden zu den Formkörpern verarbeitet werden, beispielsweise Extrudieren, Tablettieren oder durch Agglomerationsverfahren, gegebenenfalls unter Zusatz von Hilfsmitteln.

Alternativ können erfindungsgemässe Katalysatoren beispielsweise auch durch Aufbringen der Aktivkomponente auf einen Träger hergestellt werden, beispielsweise durch Beschichten oder Aufdampfen. Weiterhin können erfindungsgemässe Katalysatoren durch Verformen einer heterogenen Mischung aus Aktivkomponente oder Vorläuferverbindung hiervon mit einer Trägerkomponente oder Vorläuferverbindung hiervon erhalten werden.

Bei der erfindungsgemässen Hydrierung, bei der neben MSA andere, vorstehend definierte C₄-Dicarbonsäuren oder deren Derivate als Edukt eingesetzt werden können, wird der Katalysator in reduzierter, aktivierter Form verwendet. Die Aktivierung erfolgt mit reduzierenden Gasen, vorzugsweise Wasserstoff oder Wasserstoff/Inertgas-Gemischen, entweder vor oder nach dem Einbau in die Reaktoren, in welchen das erfindungsgemässe Verfahren durchgeführt wird. Wurde der Katalysator in oxidischer Form in den Reaktor eingebaut, so kann die Aktivierung sowohl vor dem Anfahren der Anlage mit der erfindungsgemässen Hydrierung als auch während des Anfahrens, also in situ, durchgeführt werden. Die separate Aktivierung vor dem Anfahren der Anlage erfolgt im allgemeinen mit reduzierenden Gasen, vorzugsweise Wasserstoff oder Wasserstoff/Inertgas-Gemischen bei erhöhten Temperaturen, vorzugsweise zwischen 100 und 350°C. Bei der sogenannten in-situ-Aktivierung erfolgt die Aktivierung beim Hochfahren der Anlage durch Kontakt mit Wasserstoff bei erhöhter Temperatur.

Die Katalysatoren werden vorzugsweise als Formkörper eingesetzt. Beispiele umfassen Stränge, Rippstränge, andere Extrudatformen, Tabletten, Ringe, Kugeln und Splitt. Die BET-Oberfläche der Kupferkatalysatoren sollte im oxidischen Zustand 10 bis 300 m²/g, vorzugsweise 15 bis 175 m²/g, insbesondere 20 bis 150 m²/g betragen. Die Kupferoberfläche (N₂O-Zersetzung) des reduzierten Katalysators sollte im Einbauzustand > 0,2 m²/g, vorzugsweise > 1 m²/g, insbesondere > 2 m²/g betragen.

Gemäß einer Variante der Erfindung werden Katalysatoren verwendet, die eine definierte Porosität aufweisen. Diese Katalysatoren zeigen als Formkörper ein Porenvolumen von ≥ 0,01 ml/g für Porendurchmesser > 50 nm, vorzugsweise ≥ 0,025 ml/g für Porendurchmesser > 100 nm und insbesondere ≥ 0,05 ml/g für Porendurchmesser > 200 nm. Weiterhin liegt das Verhältnis von Makroporen mit einem Durchmesser > 50 nm zum Gesamtporenvolumen für Poren mit einem Durchmesser > 4 nm bei Werten > 10 %, bevorzugt > 20 %, insbesondere > 30 %. Die erwähnten Porositäten wurden durch Quecksilber-Intrusion nach DIN 66133 bestimmt. Es wurden die Daten im Porendurchmesserbereich von 4 nm bis 300 µm ausgewertet.

Die erfindungsgemäss verwendeten Katalysatoren besitzen im allgemeinen eine ausreichende Standzeit. Für den Fall, daß die Aktivität und/oder Selektivität des Katalysators dennoch im Laufe ihrer Betriebszeit sinken sollte, kann dieser durch dem Fachmann bekannte Maßnahmen regeneriert werden. Hierzu zählt vorzugsweise eine reduktive Behandlung des Katalysators im Wasserstoffstrom bei erhöhter Temperatur. Gegebenenfalls kann der reduktiven Behandlung eine oxidative vorausgehen. Hierbei wird die Katalysatorschüttung mit einem molekularen Sauerstoff enthaltenden Gasgemisch, beispielsweise Luft, bei erhöhter Temperatur durchströmt. Weiterhin besteht die Möglichkeit, die Katalysatoren mit einem geeigneten Lösungsmittel, beispielsweise Ethanol, THF, BDO oder GBL, zu waschen und anschließend in einem Gasstrom zu trocknen.

Mit dem erfindungsgemäßen Verfahren werden Selektivitäten zu Butandiol erreicht, die generell etwas unterhalb der Selektivitäten liegen, die mit dem Verfahren nach der Anmeldung "Zweistufiges Verfahren zur Herstellung von Butandiol in zwei Reaktoren" vom 11.06.2002 der Anmelderin erreicht werden und bei dem nur sehr geringe Mengen an THF gebildet werden. Mit dem Verfahren nach der vorliegenden Anmeldung erhält man dem gegenüber immer eine deutliche Menge an THF, die - bei einer optimierten Verfahrensdurchführung - bei wenigen Gew.-% liegt. Bei einer nicht optimierten Verfahrensdurchführung - beispielsweise bei Anlegen der gleichen Temperaturen und Verwendung identischer Katalysatoren in beiden Hydrierstufen - werden bis zu 50 Gew.-% THF (bezogen auf BDO) erhalten. Die erhöhte Bildung von THF - das ja vom gewünschten Wertprodukt BDO abgetrennt werden muss - wird jedoch durch den geringen apparativen Aufwand bei der Durchführung des Verfahrens nach der vorliegenden Anmeldung ausgeglichen.

## Patentansprüche

1. Verfahren zur Herstellung von gegebenenfalls alkylsubstituiertem 1,4-Butandiol durch zweistufige katalytische Hydrierung in der Gasphase von C₄-Dicarbonsäuren und/oder deren Derivaten mit den folgenden Schritten:
a) Einführen eines Gasstroms einer C₄-Dicarbonsäure oder eines Derivates davon bei 200 bis 300°C und 10 bis 100 bar in einen ersten Reaktor oder in eine erste Reaktionszone eines Reaktors und katalytische Gasphasenhydrierung zu einem hauptsächlich gegebenenfalls alkylsubstituiertes γ-Butyrolacton enthaltenden Produkt;
b) Einführen des so erhaltenen Produktstroms in einen zweiten Reaktor oder in eine zweite Reaktionszone eines Reaktors bei einer Temperatur von 140°C bis 260°C und katalytische Gasphasenhydrierung zu gegebenenfalls alkylsubstituiertem 1,4-Butandiol,
wobei die Schritte a) und b) bei gleichem Druck durchgeführt werden;
c) Abtrennen des gewünschten Produkts von Zwischenprodukten, Nebenprodukten und gegebenenfalls nicht umgesetztem Edukt;
d) gegebenenfalls Rückführen von nicht umgesetzten Zwischenprodukten in eine oder beide Hydrierstufen,
wobei in beiden Hydrierstufen jeweils ein Katalysator verwendet wird, der ≤ 95 Gew.-%, vorzugsweise 5 bis 95 Gew.-%, insbesondere 10 bis 80 Gew.-% CuO und ≥ 5 Gew.-%, vorzugsweise 5 bis 95 Gew.-%, insbesondere 20 bis 90 Gew.-% eines oxidischen Trägers aufweist, und das der ersten Hydrierstufe entnommene Produktgemisch ohne weitere Reinigung in die zweite Hydrierstufe eingeführt wird.

2. Verfahren nach Anspruch 1, **dadurch gekennzeichnet, dass** die Eingangstemperatur in den zweiten Reaktor oder in die zweite Reaktionszone eines Reaktors niedriger ist als die Eingangstemperatur in den ersten Reaktor oder in die erste Reaktionszone.

3. Verfahren nach Anspruch 1 oder 2, **dadurch gekennzeichnet, dass** die Eingangstemperatur in den ersten Reaktor bei Werten von 235 bis 270 °C und die Eingangstemperatur in den zweiten Reaktor bei Werten von 160°C bis 225°C, insbesondere bei 180 bis 200°C, liegt.

4. Verfahren nach einem der Ansprüche 1 bis 3, **dadurch gekennzeichnet, dass** die Hot-Spot-Temperatur im ersten Reaktor bei Werten von 210 bis 310°C, vorzugsweise 245 bis 280°C liegt und das Verfahren so durchgeführt wird, dass die Hot-Spot-Temperatur oberhalb der Eintrittstemperatur und der Austrittstemperatur der Reaktionsgase liegt, wobei die Hot-Spot-Temperatur 5 bis 30°C, insbesondere 5 bis 15°C, besonders bevorzugt 5 bis 10°C, oberhalb der Eintrittstemperatur liegt.

5. Verfahren nach einem der Ansprüche 1 bis 4, **dadurch gekennzeichent, dass** sowohl in der ersten Hydrierstufe als auch in der zweiten Hydrierstufe ein Druck von 10 bis 100 bar, vorzugsweise ein Druck von 15 bis 50 bar herrscht.

6. Verfahren nach einem der Ansprüche 1 bis 5, **dadurch gekennzeichnet, dass** die Katalysatorbelastung der ersten Hydrierstufe im Bereich von 0,02 bis 1, insbesondere 0,05 bis 0,5 kg Edukt/1 Katalysator • Stunde, und die Katalysatorbelastung der zweiten Hydrierstufe im Bereich von 0,02 bis 1,5, insbesondere 0,1 bis 1 kg Edukt/1 Katalysator • Stunde, liegt

7. Verfahren nach einem der Ansprüche 1 bis 6, **dadurch gekennzeichnet, dass** das Wasserstoff/Edukt-Molverhältnis in beiden Reaktionsstufen bei Werten ≥5, vorzugsweise 20 bis 600, liegt.

8. Verfahren nach Anspruch 7, **dadurch gekennzeichnet, dass** das Wasserstoff/Edukt-Verhältnis bei der Hydrierung der ersten Stufe bei Werten von 20 bis 200, vorzugsweise 40 bis 150, insbesondere 50 bis 100, liegt.

9. Verfahren nach einem der Ansprüche 1 bis 8, **dadurch gekennzeichnet, dass** die eingesetzten Reaktoren ausgewählt sind aus der Gruppe bestehend aus Rohrreaktoren, Schachtreaktoren, Reaktoren mit innerer Wärmeabfuhr, bevorzugt Rohrbündelreaktoren, und Wirbelbettreaktoren.

10. Verfahren nach Anspruch 9, **dadurch gekennzeichnet, dass** in der ersten Hydrierstufe ein Rohrbündelreaktor eingesetzt wird.

11. Verfahren nach Anspruch 9 oder 10, **dadurch gekennzeichnet, dass** in der zweiten Hydrierstufe ein Schachtreaktor eingesetzt wird.

12. Verfahren nach einem der Ansprüche 1 bis 11, **dadurch gekennzeichnet, dass** in der ersten und/oder der zweiten Hydrierstufe mehrere Reaktoren parallel oder hintereinander geschaltet eingesetzt werden.

13. Verfahren nach einem der Ansprüche 1 bis 12, **dadurch gekennzeichnet, dass** ein Zweizonenreaktor eingesetzt wird.

14. Verfahren nach einem der Ansprüche 1 bis 13, **dadurch gekennzeichnet, dass** das Verfahren in einer Vorrichtung mit einem Hydrierkreis und einem Verdichter durchgeführt wird.

15. Verfahren nach einem der Ansprüche 1 bis 14, **dadurch gekennzeichnet, dass** das Trägermaterial des Katalysators ausgewählt ist aus der Gruppe ZnO, Al₂O₃, SiO₂, TiO₂, ZrO₂, CeO₂, MgO, CaO, SrO, BaO und Mn₂O₃ und Mischungen davon, vorzugsweise aus der Gruppe ZnO/Al₂O₃-Gemische, delta-, theta-, alpha- und eta-Modifikationen des Al₂O₃ sowie Mischungen, die mindestens eine Komponente aus der Gruppe SiO₂, TiO₂, ZrO₂ einerseits und aus der Gruppe ZnO, MgO, CaO, SrO und BaO andererseits enthalten.

16. Verfahren nach einem der Ansprüche 1 bis 15, **dadurch gekennzeichnet, dass** das Trägermaterial ausgewählt ist aus ZnO, ZnO/Al₂O₃-Gemischen im Gewichtsverhältnis 100:1 bis 1:2 und Mischungen von SiO₂ mit MgO, CaO und/oder ZnO im Gewichtsverhältnis 200:1 bis 1:1.

17. Verfahren nach einem der Ansprüche 1 bis 16, **dadurch gekennzeichnet, dass** der Katalysator ein oder mehrere weitere Metalle, vorzugsweise Pd, oder eine Verbindung eines oder mehrer weiterer Metalle, vorzugsweise ein Oxid, aus den Gruppen 1 bis 14 des Periodensystems der Elemente enthält.

18. Verfahren nach einem der Ansprüche 1 bis 14, **dadurch gekennzeichnet, dass** der Katalysator als Formkörper eingesetzt wird, vorzugsweise in Form von Strängen, Rippsträngen, Tabletten, Ringen, Kugeln oder Splitt.

19. Verfahren nach einem der Ansprüche 1 bis 18, **dadurch gekennzeichnet, dass** die BET-Oberfläche der Kupferkatalysatoren im oxidischen Zustand 10 bis 300 m²/g, vorzugsweise 15 bis 175 m²/g, insbesondere 20 bis 150 m²/g beträgt.

20. Verfahren nach einem der Ansprüche 1 bis 19, **dadurch gekennzeichnet, dass** die Kupferoberfläche des reduzierten Katalysators im Einbauzustand > 0,2 m²/g, vorzugsweise > 1 m²/g, insbesondere > 2 m²/g, beträgt.

21. Verfahren nach einem der Ansprüche 1 bis 20, **dadurch gekennzeichnet, dass** die im ersten und zweiten Reaktor verwendeten Katalysatoren gleich oder voneinander verschieden sind, vorzugsweise voneinander verschieden sind.

22. Verfahren nach einem der Ansprüche 1 bis 21, **dadurch gekennzeichnet, dass** der eingesetzte Katalysator als Formkörper ein Porenvolumen von ≥ 0,01 ml/g für Porendurchmesser > 50 nm, vorzugsweise ≥ 0,025 ml/g für Porendurchmesser > 100 nm und insbesondere ≥ 0,05 ml/g für Porendurchmesser > 200 nm, aufweist.

23. Verfahren nach einem der Ansprüche 1 bis 12, **dadurch gekennzeichnet, dass** der eingesetzte Katalysator als Formkörper ein Verhältnis von Makroporen mit einem Durchmesser > 50 nm zum Gesamtporenvolumen für Poren mit einem Durchmesser > 4 nm mit Werten > 10 %, bevorzugt > 20 %, insbesondere > 30 %, aufweist.

24. Verfahren nach einem der Ansprüche 1 bis 23, **dadurch gekennzeichnet, daß** Maleinsäureanhydrid als Edukt in die Reaktion eingesetzt wird.

25. Verfahren nach einem der Ansprüche 1 bis 24, **dadurch gekennzeichnet, daß** Maleinsäureanhydrid eingesetzt wird, das durch Oxidation von Benzol, C₄-Olefinen oder n-Butan hergestellt wurde, wobei das durch Oxidation erhaltene Roh-Maleinsäureanhydrid mit einem Lösungsmittel (Absorptionsmittel) aus dem Rohproduktgemisch extrahiert und anschließend aus diesem Lösungsmittel mit Wasserstoff ausgetrieben wurde.

26. Verfahren nach einem der Ansprüche 1 bis 25, **dadurch gekennzeichnet, daß** das Absorptionsmittel ausgewählt ist aus der Gruppe bestehend aus Trikresylphosphat, Dibutylinaleat, hochmolekularen Wachsen, aromatischem Kohlenwasserstoff mit einem Molekulargewicht zwischen 150 und 400 und einem Siedepunkt oberhalb 140 °C, vorzugsweise Dibenzol, Di-C₁-C₄-Alkylestern aromatischer und aliphatischer Dicarbonsäuren, vorzugsweise Dimethyl-2,3-NaphthalinDicarbonsäure und/oder Dimethyl-1,4-Cyclohexan-Dicarbonsäure, Methylestern langkettiger Fettsäuren mit 14 bis 30 Kohlenstoffatomen, hochsiedenden Ethern, vorzugsweise Dimethylether von Polyethylenglykol, vorzugsweise von Tetraethylenglykol, und Alkylphthalaten und Dialkylphthalaten mit C₁-C₁₈-Alkylgruppen, vorzugsweise aus der Gruppe Dimethylphthalat, Diethylphthalat, Dibutylphthalat, Di-n-Propyl- und Di-iso-Propylphthalat, Undecylphthalat, Diundecylphthalat, Methylphthalat, Ethylphthalat, Butylphthalat, n-Propyl- und iso-Propylphthalat.

27. Verfahren nach einem der Ansprüche 1 bis 26, **dadurch gekennzeichnet, daß** das Maleinsäureanhydrid aus dem Absorptionsmittel im Vakuum oder bei Drücken, die dem Druck der Hydrierung entsprechen oder maximal 10 % oberhalb dieses Druckes liegen, ausgetrieben wird.

28. Verfahren nach einem der Ansprüche 1 bis 27, **dadurch gekennzeichnet, dass** das Verfahren diskontinuierlich, semi-kontinuierlich oder kontinuierlich durchgeführt wird, vorzugsweise kontinuierlich.

## Claims

1. A process for preparing optionally alkyl-substituted 1,4-butanediol by two-stage catalytic hydrogenation in the gas phase of C₄-dicarboxylic acids and/or of derivatives thereof having the following steps:
a) introducing a gas stream of a C₄-dicarboxylic acid or of a derivative thereof at from 200 to 300°C and from 10 to 100 bar into a first reactor or into a first reaction zone of a reactor and catalytically hydrogenating it in the gas phase to a product which comprises mainly optionally alkyl-substituted γ-butyrolactone;
b) introducing the product stream thus obtained into a second reactor or into a second reaction zone of a reactor at a temperature of from 140°C to 260°C and catalytically hydrogenating it in the gas phase to optionally alkyl-substituted 1,4-butanediol;
steps a) and b) being carried out at the same pressure;
c) removing the desired product from intermediates, by-products and any unconverted reactant;
d) optionally recycling unconverted intermediates into one or both hydrogenation stages,
said hydrogenation stages each using a catalyst which comprises ≤ 95% by weight, preferably from 5 to 95% by weight, in particular from 10 to 80% by weight, of CuO, and ≥ 5% by weight, preferably from 5 to 95% by weight, in particular from 20 to 90% by weight, of an oxidic support, and the product mixture withdrawn from the first hydrogenation stage being introduced into the second hydrogenation stage without further purification.

2. The process according to claim 1, wherein the entrance temperature into the second reactor or into the second reaction zone of a reactor is lower than the entrance temperature into the first reaction zone.

3. The process according to claim 1 or 2, wherein the entrance temperature into the first reactor is from 235 to 270°C and the entrance temperature into the second reactor is from 160°C to 225°C, in particular from 180 to 200°C.

4. The process according to any of claims 1 to 3, wherein the hot spot temperature in the first reactor is from 210 to 310°C, preferably from 245 to 280°C, and the process is carried out in such a manner that the hot spot temperature is above the entrance temperature and the exit temperature of the reaction gases, and is from 5 to 30°C, in particular from 5 to 15°C, more preferably from 5 to 10°C, above the entrance temperature.

5. The process according to any of claims 1 to 4, wherein the pressure both in the first hydrogenation stage and in the second hydrogenation stage is from 10 to 100 bar, preferably from 15 to 50 bar.

6. The process according to any of claims 1 to 5, wherein the catalyst space velocity of the first hydrogenation stage is in the range from 0.02 to 1, in particular from 0.05 to 0.5, kg of reactant/1 catalyst • hour, and the catalyst space velocity of the second hydrogenation stage is in the range from 0.02 to 1.5, in particular from 0.1 to 1, kg of reactant/1 of catalyst • hour.

7. The process according to any of claims 1 to 6, wherein the hydrogen/reactant molar ratio in both reaction stages is ≥ 5, preferably from 20 to 600.

8. The process according to claim 7, wherein the hydrogen/reactant ratio in the first stage hydrogenation is from 20 to 200, preferably from 40 to 150, in particular from 50 to 100.

9. The process according to any of claims 1 to 8, wherein the reactors used are selected from the group consisting of tubular reactors, shaft reactors, reactors having internal heat removal means, preferably tube bundle reactors, and fluidized bed reactors.

10. The process according to claim 9, wherein a tube bundle reactor is used in the first hydrogenation stage.

11. The process according to claim 9 or 10, wherein a shaft reactor is used in the second hydrogenation stage.

12. The process according to any of claims 1 to 11, wherein more than one reactor connected in parallel or in series is used in the first and/or second hydrogenation stage.

13. The process according to any of claims 1 to 12, wherein a two-zone reactor is used.

14. The process according to any of claims 1 to 13, which is carried out in an apparatus comprising a hydrogenation circuit and a compressor.

15. The process according to any of claims 1 to 14, wherein the support material of the catalyst is selected from the group of ZnO, Al₂O₃, SiO₂, TiO₂, ZrO₂, CeO₂, MgO, CaO, SrO, BaO and Mn₂O₃ and mixtures thereof, preferably from the group of ZnO/Al₂O₃ mixtures, the delta-, theta-, alpha-and eta-modifications of Al₂O₃ and also mixtures which comprise at least one component each firstly from the group of SiO₂, TiO₂, ZrO₂, and secondly from the group of ZnO, MgO, CaO, SrO and BaO.

16. The process according to any of claims 1 to 15, wherein the support material is selected from ZnO, ZnO/Al₂O₃ mixtures in a weight ratio of from 100:1 to 1:2 and mixtures of SiO₂ with MgO, CaO and/or ZnO in a weight ratio of 200:1 to 1:1.

17. The process according to any of claims 1 to 16, wherein the catalyst comprises one or more further metals, preferably Pd, or a compound of one or more further metals, preferably an oxide, from groups 1 to 14 of the Periodic Table.

18. The process according to any of claims 1 to 14, wherein the catalyst is used in the form of shaped bodies, preferably in the form of extrudates, ribbed extrudates, tablets, rings, spheres or spall.

19. The process according to any of claims 1 to 18, wherein the BET surface area of the copper catalysts in the oxidic state is from 10 to 300 m²/g, preferably from 15 to 175 m²/g, in particular from 20 to 150 m²/g.

20. The process according to any of claims 1 to 19, wherein the copper surface area of the reduced catalyst in the installed state is > 0.2 m²/g, preferably > 1 m²/g, in particular > 2 m²/g.

21. The process according to any of claims 1 to 20, wherein the catalysts used in the first and second reactors are identical or different, preferably different.

22. The process according to any of claims 1 to 21, wherein the shaped bodies of the catalyst used have a pore volume of ≥ 0.01 ml/g for pore diameters of > 50 nm, preferably of ≥ 0.025 ml/g for pore diameters of > 100 nm and in particular of ≥ 0.05 ml/g for pore diameters of > 200 nm.

23. The process according to any of claims 1 to 12, wherein the shaped bodies of the catalyst used have a ratio of macropores having a diameter of > 50 nm to the total pore volume for pores having a diameter of > 4 nm of > 10%, preferably > 20%, in particular > 30%.

24. The process according to any of claims 1 to 23, wherein the reactant used in the reaction is maleic anhydride.

25. The process according to any of claims 1 to 24, wherein maleic anhydride is used which has been prepared by oxidizing benzene, C₄-olefins or n-butane, and the crude maleic anhydride obtained by oxidation has been extracted from the crude product mixture using a solvent absorbent and then stripped from this solvent using hydrogen.

26. The process according to any of claims 1 to 25, wherein the absorbent is selected from the group consisting of tricresyl phosphate, dibutyl maleate, high molecular weight waxes, aromatic hydrocarbon having a molecular weight of from 150 to 400 and a boiling point above 140°C, preferably dibenzene, di-C₁-C₄-alkyl esters of aromatic and aliphatic dicarboxylic acids, preferably dimethyl-2,3-naphthalenedicarboxylate and/or dimethyl-1,4-cyclohexanedicarboxylate, methyl esters of long-chain fatty acids having from 14 to 30 carbon atoms, high-boiling ethers, preferably dimethyl ethers of polyethylene glycol, preferably of tetraethylene glycol, and alkyl phthalates and dialkyl phthalates having C₁-C₁₈-alkyl groups, preferably from the group of dimethyl phthalate, diethyl phthalate, dibutyl phthalate, di-n-propyl and diiso-propyl phthalate, undecyl phthalate, diundecyl phthalate, methyl phthalate, ethyl phthalate, butyl phthalate, n-propyl and iso-propyl phthalate.

27. The process according to any of claims 1 to 26, wherein the maleic anhydride is stripped from the absorbent under reduced pressure or pressures which correspond to the hydrogenation pressure or are a maximum of 10% above this pressure.

28. The process according to any of claims 1 to 27, which is carried out batchwise, semicontinuously or continuously, preferably continuously.

## Revendications

1. Procédé de fabrication de 1,4-butanediol éventuellement à substitution alkyle par hydrogénation catalytique à deux étapes en phase gazeuse d'acides dicarboxyliques en C₄ et/ou leurs dérivés, selon les étapes suivantes :
a) introduction d'un courant gazeux d'un acide dicarboxylique en C₄ ou d'un de ses dérivés à 200 à 300 °C et 10 à 100 bar dans un premier réacteur ou dans une première zone de réaction d'un réacteur et hydrogénation catalytique en phase gazeuse pour former un produit contenant principalement de la γ-butyrolactone éventuellement à substitution alkyle ;
b) introduction du courant de produit ainsi obtenu dans un second réacteur ou dans une seconde zone de réaction d'un réacteur à une température de 140 °C à 260 °C et hydrogénation catalytique en phase gazeuse pour former du 1,4-butanediol éventuellement à substitution alkyle,
les étapes a) et b) étant réalisées à la même pression ;
c) séparation du produit souhaité des produits intermédiaires, des produits secondaires et éventuellement du réactif non réagi ;
d) éventuellement recyclage des produits intermédiaires non réagis dans une étape d'hydrogénation ou les deux,
un catalyseur qui comporte ≤ 95 % en poids, de préférence 5 à 95 % en poids, notamment 10 à 80 % en poids, de CuO et ≥ 5 % en poids, de préférence 5 à 95 % en poids, notamment 20 à 90 % en poids, d'un support oxydique étant utilisé à chaque fois dans les deux étapes d'hydrogénation, et le mélange de produits soutiré de la première étape d'hydrogénation étant introduit sans purification supplémentaire dans la seconde étape d'hydrogénation.

2. Procédé selon la revendication 1, **caractérisé en ce que** la température d'entrée dans le second réacteur ou dans la seconde zone de réaction d'un réacteur est inférieure à la température d'entrée dans le premier réacteur ou dans la première zone de réaction.

3. Procédé selon la revendication 1 ou 2, **caractérisé en ce que** la température d'entrée dans le premier réacteur se situe à des valeurs de 235 à 270 °C et la température d'entrée dans le second réacteur à des valeurs de 160 °C à 225 °C, notamment 180 à 200 °C.

4. Procédé selon l'une quelconque des revendications 1 à 3, **caractérisé en ce que** la température d'un point chaud dans le premier réacteur se situe à des valeurs de 210 à 310 °C, de préférence 245 à 280 °C, et le procédé est réalisé de manière à ce que la température du point chaud se situe au-dessus de la température d'entrée et de la température de sortie des gaz réactionnels, la température du point chaud se situant 5 à 30 °C, notamment 5 à 15 °C, de manière particulièrement préférée 5 à 10 °C, au-dessus de la température d'entrée.

5. Procédé selon l'une quelconque des revendications 1 à 4, **caractérisé en ce qu'**une pression de 10 à 100 bar, de préférence une pression de 15 à 50 bar, règne aussi bien dans la première étape d'hydrogénation que dans la seconde étape d'hydrogénation.

6. Procédé selon l'une quelconque des revendications 1 à 5, **caractérisé en ce que** la charge du catalyseur de la première étape d'hydrogénation se situe dans la plage allant de 0,02 à 1, notamment 0,05 à 0,5 kg de réactif/1 de catalyseur-heure, et la charge du catalyseur de la seconde étape d'hydrogénation dans la plage allant de 0,02 à 1,5, notamment 0,1 à 1 kg de réactif/1 de catalyseur·heure.

7. Procédé selon l'une quelconque des revendications 1 à 6, **caractérisé en ce que** le rapport molaire hydragène/réactif se situe à des valeurs ≥ 5, de préférence de 20 à 600, dans les deux étapes de réaction.

8. Procédé selon la revendication 7, **caractérisé en ce que** le rapport hydrogène/réactif se situe à des valeurs de 20 à 200, de préférence de 40 à 150, notamment de 50 à 100, lors de l'hydrogénation de la première étape.

9. Procédé selon l'une quelconque des revendications 1 à 8, **caractérisé en ce que** les réacteurs utilisés sont choisis dans le groupe constitué des réacteurs tubulaires, des réacteurs à puits, des réacteurs à dissipation de chaleur interne, de préférence des réacteurs à faisceau de tubes, et des réacteurs à lit fluidisé.

10. Procédé selon la revendication 9, **caractérisé en ce qu'**un réacteur à faisceau de tubes est utilisé dans la première étape d'hydrogénation.

11. Procédé selon la revendication 9 ou 10, **caractérisé en ce qu'**un réacteur à puits est utilisé dans la seconde étape d'hydrogénation.

12. Procédé selon l'une quelconque des revendications 1 à 11, **caractérisé en ce que** plusieurs réacteurs sont utilisés en parallèle ou connectés les uns après les autres dans la première et/ou la seconde étape d'hydrogénation.

13. Procédé selon l'une quelconque des revendications 1 à 12, **caractérisé en ce qu'**un réacteur à deux zones est utilisé.

14. Procédé selon l'une quelconque des revendications 1 à 13, **caractérisé en ce que** le procédé est réalisé dans un dispositif muni d'un cycle d'hydrogénation et d'un compresseur.

15. Procédé selon l'une quelconque des revendications 1 à 14, **caractérisé en ce que** le matériau support du catalyseur est choisi dans le groupe du ZnO, Al₂O₃, SiO₂, TiO₂, ZrO₂, CeO₂, MgO, CaO, SrO, BaO et Mn₂O₃ et leurs mélanges, de préférence dans le groupe des mélanges ZnO/Al₂O₃, des formes delta, thêta, alpha et êta d'Al₂O₃, ainsi que des mélanges qui contiennent au moins un composant du groupe SiO₂, TiO₂, ZrO₂ d'une part et du groupe ZnO, MgO, CaO, SrO et BaO d'autre part.

16. Procédé selon l'une quelconque des revendications 1 à 15, **caractérisé en ce que** le matériau support est choisi parmi ZnO, les mélanges ZnO/Al₂O₃ ayant un rapport en poids de 100:1 à 1:2 et les mélanges de SiO₂ avec MgO, CaO et/ou ZnO ayant un rapport en poids de 200:1 à 1:1.

17. Procédé selon l'une quelconque des revendications 1 à 16, **caractérisé en ce que** le catalyseur contient un ou plusieurs métaux supplémentaires, de préférence Pd, ou un composé d'un ou de plusieurs métaux supplémentaires, de préférence un oxyde, des groupes 1 à 14 du tableau périodique des éléments.

18. Procédé selon l'une quelconque des revendications 1 à 14, **caractérisé en ce que** le catalyseur est utilisé sous la forme d'un corps moulé, de préférence sous la forme de filaments, de filaments rainurés, de tablettes, d'anneaux, de billes ou de copeaux.

19. Procédé selon l'une quelconque des revendications 1 à 18, **caractérisé en ce que** la surface BET des catalyseurs de cuivre à l'état oxydique est de 10 à 300 m²/g, de préférence 15 à 175 m²/g, notamment 20 à 150 m²/g.

20. Procédé selon l'une quelconque des revendications 1 à 19, **caractérisé en ce que** la surface de cuivre du catalyseur réduit à l'état monté est > 0,2 m²/g, de préférence > 1 m²/g, notamment > 2 m²/g.

21. Procédé selon l'une quelconque des revendications 1 à 20, **caractérisé en ce que** les catalyseurs utilisés dans le premier et le second réacteur sont identiques ou différents l'un de l'autre, de préférence différents l'un de l'autre.

22. Procédé selon l'une quelconque des revendications 1 à 21, **caractérisé en ce que** le catalyseur utilisé sous la forme d'un corps moulé présente un volume poreux ≥ 0,01 ml/g pour un diamètre de pore > 50 nm, de préférence ≥ 0,025 ml/g pour un diamètre de pore > 100 nm et notamment ≥ 0,05 ml/g pour un diamètre de pore > 200 nm.

23. Procédé selon l'une quelconque des revendications 1 à 12, **caractérisé en ce que** le catalyseur utilisé sous la forme d'un corps moulé présente un rapport entre les macropores ayant un diamètre > 50 nm et le volume poreux total des pores ayant un diamètre > 4 nm à des valeurs > 10 %, de préférence > 20 %, notamment > 30 %.

24. Procédé selon l'une quelconque des revendications 1 à 23, **caractérisé en ce que** de l'anhydride de l'acide maléique est utilisé en tant que réactif dans la réaction.

25. Procédé selon l'une quelconque des revendications 1 à 24, **caractérisé en ce que** de l'anhydride de l'acide maléique, qui a été fabriqué par oxydation de benzène, d'oléfines en C₄ ou de n-butane, est utilisé, l'anhydride de l'acide maléique brut obtenu par oxydation étant extrait du mélange de produits bruts avec un solvant (agent d'absorption), puis débarrassé de ce solvant avec de l'hydrogène.

26. Procédé selon l'une quelconque des revendications 1 à 25, **caractérisé en ce que** l'agent d'absorption est choisi dans le groupe constitué du phosphate de tricrésyle, du maléate de dibutyle, des cires de poids moléculaire élevé, d'un hydrocarbure aromatique ayant un poids moléculaire compris entre 150 et 400 et un point d'ébullition supérieur à 140 °C, de préférence le dibenzène, des esters dialkyliques en C₁-C₄ d'acides dicarboxyliques aromatiques et aliphatiques, de préférence l'acide diméthyl-2,3-naphtaline-dicarboxylique et/ou l'acide diméthyl-1,4-cyclohexane-dicarboxylique, des esters méthyliques d'acides gras à longue chaîne contenant 14 à 30 atomes de carbone, des éthers de point d'ébullition élevé, de préférence des éthers diméthyliques de polyéthylène glycol, de préférence de tétraéthylène glycol, et des phtalates d'alkyle et des phtalates de dialkyle contenant des groupes alkyle en C₁-C₁₈, de préférence du groupe du phtalate de diméthyle, du phtalate de diéthyle, du phtalate de dibutyle, du phtalate de di-n-propyle et de di-iso-propyle, du phtalate d'undécyle, du phtalate de di-undécyle, du phtalate de méthyle, du phtalate d'éthyle, du phtalate de butyle, du phtalate de n-propyle et d'iso-propyle.

27. Procédé selon l'une quelconque des revendications 1 à 26, **caractérisé en ce que** l'anhydride de l'acide maléique est débarrassé de l'agent d'absorption sous vide ou à des pressions qui correspondent à la pression de l'hydrogénation ou qui se situent au plus 10 % au-dessus de cette pression.

28. Procédé selon l'une quelconque des revendications 1 à 27, **caractérisé en ce que** le procédé est réalisé de manière discontinue, semi-continue ou continue, de préférence continue.
